# EUROPEAN PATENT SPECIFICATION

(11) **EP 2 786 704 B1**
(45) Date of publication and mention of the grant of the patent: **05.10.2016**
(21) Application number: 13001675.1
(22) Date of filing: 02.04.2013
(51) Int. Cl.: A61B 5/0205, A61B 5/021, A61B 5/04, G06F 19/00, A61B 5/00, A61B 5/0476, A61B 5/0488, A61B 5/0456

(54) **Device and method for assessing mortality risk of a cardiac patient**
Vorrichtung und Verfahren zur Beurteilung des Sterberisikos bei Herzpatienten
Dispositif et procédé pour évaluer le risque de mortalité d'un patient cardiaque

(43) Date of publication of application: 08.10.2014
(73) Proprietor: Schmidt, Georg, 81675 München (DE)
(72) Inventor: Schmidt, Georg, 81675 München (DE)
(74) Representative: Grünecker Patent- und Rechtsanwälte PartG mbB

(56) References cited:
- WO-A1-89/09022
- WO-A1-2010/139859
- US-A1- 2011 301 479
- RABKIN S W ET AL: "Relationship of ventricular ectopy in men without apparent heart disease to occurrence of ischemic heart disease and sudden death", AMERICAN HEART JOURNAL, MOSBY- YEAR BOOK INC, US, vol. 101, no. 2, 1 February 1981 (1981-02-01), pages 135-142, XP022945029, ISSN: 0002-8703, DOI: 10.1016/0002-8703(81)90655-4 [retrieved on 1981-02-01]
- VOSS A ET AL: "Postextrasystolic regulation patterns of blood pressure and heart rate in patients with idiopathic dilated cardiomyopathy", JOURNAL OF PHYSIOLOGY (CAMBRIDGE), vol. 538, no. 1, 1 January 2002 (2002-01-01), pages 271-278, XP008164247, ISSN: 0022-3751, DOI: 10.1113/jphysiol.2001.013044

## Description

### FIELD OF INVENTION

The invention relates to a device and method for assessing a mortality risk of a cardiac patient based on at least one vital sign or biosignal of said patient.

### BACKGROUND

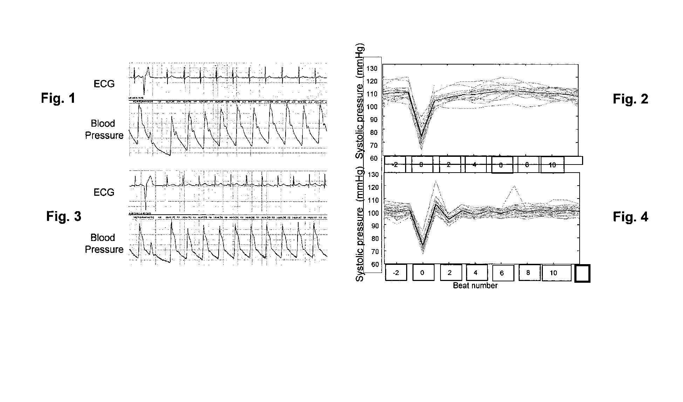

The document US 2011/301479 A1 **(D1**) relates to a system and method for assessing a likelihood of a patient to experience a cardiac arrhythmia using dynamic changes in a biological parameter. In various embodiments, a risk stratification algorithm considers a T-wave alternans marker (412, Fig. 4) which determines the maximum difference in the ST-T windows between two modified moving average templates constructed from alternate beats. A weighted moving average is applied to limit the effect of artifacts and the contributions of single beats (see section [0082]). In an embodiment described in section [0083], if the absolute value of the difference in measured Tpeak values following premature ventricular contraction 86 is less than the threshold, compensatory pause and, in an embodiment, an abnormal autonomic reflex, then patient may be identified as not having significant T-wave alternans and, as a result, as being at higher risk of future arrhythmia.
The document WO 2010/139859 A1 **(D2)** discloses an apparatus, a method, and a computer program for predicting a risk to a cardiac death. The apparatus (100) comprises a processor (116) configured to calculate morphological parameter values (values of total cosine R-to-T, values of QRS complex loop asymmetry, values of T-wave loop dispersion, values of QRS complex and T-wave principal component analysis, values of an angle between a main QRS complex loop vector and a main T-wave loop vector) of QRS complex and T wave from a sample period within electrocardiography data recorded from a subject.
The document WO 89/09022 A1 **(D3)** discloses a device for determining the probability of death in cardiovascular patients including an electrocardiograph adapted to deliver a signal in the form of an electrical waveform containing information about the condition of the patient's heart; a waveform recognition and measurement device adapted to analyze the waveform and generate output based on the analysis; and a computer adapted to receive the output and calculate a numerical value representing the probability based on the output. Also provided is a method for assessing cardiovascular mortality risk at a health care facility or provider using this device.

### SUMMARY

Prognostic scores for assessing a mortality risk of a cardiac patient such as LVEF or the GRACE score according to Eagle et al. (Eagle KA, Lim MJ, Dabbous OH, et al. "A validated prediction model for all forms of acute coronary syndrome: estimating the risk of 6-month postdischarge death in an international registry", J Am Med Assoc 2004; 291:2727-33) are well known in the prior art. The elements of the GRACE score include patient-related information such as the age, history of past heart failure, history of myocardial infarction, serum creatinine at admission, cardiac biomarkers status at admission, SBP at admission, pulse at admission, ST deviation at admission, and in-hospital percutaneous coronary intervention. However, mortality risk assessment of cardiac patients such as myocardial infarction patients according to the GRACE score or the LVEF leaves room for improvement regarding the quality of the risk assessment.

For risk assessment in various cardiac conditions, an improved patient selection for ICD implantation, for cardiac resynchronization therapy, and a contribution to the optimization of heart failure therapy, it is desirable to improve the precision of mortality risk assessment of a cardiac patient.

The object of the invention is, therefore, to provide a device and method for assessing mortality risk of a cardiac patient with improved precision (at the cost of only minor additional effort) as compared to conventional mortality risk assessment concepts for cardiac patients, in particular for myocardial infarction patients.

To solve the above object, the invention provides, according to a first inventive aspect, a device for assessing a mortality risk of a cardiac patient based on at least one vital sign or biosignal of said patient, said device comprising:
- A data provision unit being configured to provide at least one data function of at least one vital sign or biosignal of said patient.
- A data processing unit being configured to process data of said at least one data function for assessing a mortality risk of said patient by performing the following actions:
   Selecting at least one data sequence from said at least one data function according to a predetermined routine.
      ▪ Computing a specific behavior of at least one parameter of said at least one data function based on said at least one selected data sequence.
      ▪ Assessing a mortality risk of said patient based on said computed behavior.

The term "specific behaviour" is meant to comprise any information which can be taken from any parameter of said at least one data function in relation to other parameters of said at least one data function. It is preferably a quotient indicating a physiological response of said patient to a specific cardial activity such as on VPC.

The invention is substantially based on the seminal finding that a specific physiological behavior of a cardiac patient may be used for assessing a mortality risk in (post-) myocardial infarction (MI) patients. More specifically, it has been found that a post-extrasystolic potentiation (PESP) and/or a post-extrasystolic T-wave change (PEST), in particular when following a premature ventricular contraction (or ventricular premature complex (VPC) or ventricular extrasystole (VES)), reflects a strong and independent mortality risk predictor in (post-) myocardial infarction patients.

PESP is the pulse wave augmentation after a VPC. It relates to cellular calcium homeostasis and is pronounced in heart failure patients, as will be explained further down. This ability of PESP to predict mortality in myocardial infarction (MI) survivors has been successfully tested in studies.

The physiological basis of PESP has been investigated previously, and various concepts have been developed. It is assumed that the observed PESP pattern consists of a myocardial and a vascular component. The myocardial component is based on myocyte calcium handling: The premature beat leads to a reduced amount of calcium released from the sarcoplasmic reticulum. The resulting smaller calcium transient leads to increased systolic calcium influx through L-type calcium channels and decreased diastolic calcium extrusion through the sodium-calcium exchanger. More calcium is accumulated in sarcoplasmic reticulum during the post-extrasystolic pause and is available to be released during the next contraction. Accordingly, contractile force of the post-extrasystolic beat is elevated. The observed blood pressure pattern is further influenced by the vascular compliance and the decline of the diastolic blood pressure during the postextrasystolic pause.

### PESP in Heart Failure

PESP in heart failure patients observed in clinical studies reflects a strong risk predictor in post-infarction patients. The following hypothesis, based on both altered myocardial and vascular alterations, might be proposed explaining the presence of PESP in heart failure: In the failing myocardium, the capacity of the sarcoplasmic reticulum to accumulate calcium is reduced due to reduced sarcoplasmic reticulum calcium uptake, increased leak through sarcoplasmic reticulum calcium release channels (ryanodine receptors) and increased expression of the sodium-calcium exchanger. As a consequence, while in the non-failing myocardium, 30% of cycling calcium originates from calcium influx through L-type calcium channels and efflux through the sodium-calcium exchanger, this fraction increases up to 50% in heart failure with a reduction in recirculation fraction. The post-extrasystolic pause allows for a better calcium accumulation in the sarcoplasmic reticulum from higher diastolic calcium levels. In addition, reverse mode sodium-calcium exchange may contribute to sarcoplasmic reticulum calcium loading in failing myocardium.

The vascular component may result from increased peripheral resistance with a smaller decrease in diastolic aortic pressure during the post-extrasystolic pause which may allow for higher systolic pressure in the subsequent beat of augmented contraction. The proposed myocardial mechanism for PESP may directly explain higher mortality risk. Increased expression of the sodium-calcium exchanger may create either calcium overload in the reversed mode or net inward current in the forward mode. While calcium influx in the reversed mode would cause early afterdepolarizations, forward mode calcium exchange would induce delayed afterdepolarizations. Both early and delayed afterdepolarizations increase the risk of arrhythmias.

### PESP and the prognostic role of ventricular ectopic beats

The findings from clinical studies also advance the understanding of the predictive value of ventricular ectopic frequency in post-MI patients. Frequent VPCs have so far been perceived as arrhythmia triggers, independent of pro-arrhythmic substrate and modulators, such as impaired cardiac autonomic status. This concept is challenged by the finding that frequent VPCs leading to PESP signify very different prognosis from similarly frequent VPCs without PESP. If, as already described, PESP reflects reduction of trans-sarcoplasmic reticulum calcium currents at the expense of increased trans-plasmamembrane fluxes, these changes might lead to instabilities facilitating the development of ventricular tachyarrhythmias. Thus, patients with PESP and frequent VPCs might be not only at risk of death due to advanced heart failure, but also at increased risk of sudden cardiac death due to sustained ventricular arrhythmias both triggered by VPCs and also facilitated by the calcium handling abnormalities.

To make the calculation less noise sensitive, it is proposed to relate the first post-VPC pulse wave amplitude to the mean of the subsequent nine pulse wave amplitudes rather than to the pre-VPC pulse wave amplitude. However, when using the ratio between the post- and pre-VPC pulse wave amplitudes, the results are practically the same although reaching slightly lesser statistical significances.

### Potential clinical applications

Assessment of PESP is quantified by analysis of the blood pressure response to VPCs and requires no particular expenditure; PESP can be observed either after spontaneous VPCs or after instrumentally induced VPCs. In this way, programmed ventricular stimulation might also evaluate abnormalities of myocardial calcium cycling. A scale of potential clinical applications may be envisioned, including risk assessment in various cardiac conditions, an improved patient selection for cardiac resynchronization therapy, and a contribution to the optimization of heart failure therapy.

Preferably, said data provision unit is configured to perform at least one of the following actions:
- Recording said at least one vital sign or biosignal, preferably in digital form.
- Recording said at least one vital sign or biosignal continuously for a predetermined period of time, preferably for at least 30 minutes.
- Recording said at least one vital sign or biosignal with non-invasive recording means, preferably using at least an electrocardiogram recorder, preferably a high resolution electrocardiogram recorder with at least 1.6 kHz in orthogonal XYZ leads, preferably for continuously recording an electrocardiogram of said patient.
- Recording said at least one vital sign or biosignal in a resting position of said patient, preferably in a supine resting position of said patient.
- Recording at least two different vital signs or biosignals simultaneously, preferably an electrocardiogram and blood pressure, preferably continuous arterial blood pressure.
- Storing the recorded data in data storage means, preferably in digital form.
- Providing said at least one data function as a function of said at least one vital sign or biosignal of said patient over the time.
- Enabling verification and/or review and/or manual correction of said at least one data function, preferably including the elimination of artefacts, more preferably enabling review and/or manual correction of QRS classifications so as to differentiate sinus and ventricular premature complexes (VPC).
- Providing at least two different data functions from simultaneous recordings of at least two different vital signs or biosignals, preferably simultaneous recordings of electrocardiogram and blood pressure.
- Storing said at least one data function in data storage means, preferably in digital form.
- Loading said at least one data function from data storage means, preferably in digital form.

The occurrence of a specific cardiac activity and a specific physiological response may be calculated on the basis of various data functions of at least one vital sign or biosignal of said patient, such as an ECG and/or systolic arterial blood pressure. An ECG is the preferred way to measure and diagnose abnormal rhythms of the heart.

According to the invention, mortality risk assessment for a cardiac patient can be automated and performed within e.g. only 30 minutes using only standard clinical equipment. Moreover, mortality risk assessment according to the invention can be performed remotely from the cardiac patient using only the at least one data function of at least one previously recorded vital sign or biosignal of said patient as an input. The inventive concept significantly improves the quality of risk assessment for said cardiac patient as compared to the conventional mortality risk assessment concepts. The output of the mortality risk assessment according to the invention may be a mortality risk predictor for said cardiac patient within five years after myocardial infarction, such as the number of counted VPCs or variables representing PESP or PEST.

Simultaneous recordings of ECG and systolic arterial blood pressure are generally preferred data functions. It is desirable that the data provision unit provides access to the raw recording signals and eliminates artefacts where needed. It is further desirable that the ECG can be reviewed and manually corrected as appropriate in case of heart beat annotations.

The information on blood pressure in response to a premature ventricular contraction may be derived from a noninvasive continuous recording of arterial blood pressure over the time and can also be visualized e.g. by providing a graph of said data function. Preferably, the continuous arterial blood pressure is simultaneously recorded with an ECG, so that the timing of a premature ventricular contraction calculated from a data function of an ECG can easily be transferred to the data function of blood pressure for the subsequent calculation of the blood pressure variability in response to the premature ventricular contraction. A routine may be computer-implemented and executed by the data processing unit in order to automate the selection of relevant data sequences from the data functions and for computing the specific behavior of the at least one data function parameter based on the selected data sequences according to known mathematic considerations.

Further preferably, said data processing unit is configured to select said at least one data sequence from said at least one data function by performing at least one of the following actions:
- Identifying specific patterns within said at least one data function, preferably QRS-complexes of an electrocardiogram.
- Identifying data points of said at least one data function correlating with at least one of the following:
   ▪ Cardiac activities, preferably cardiac activities of the same kind
   ▪ R-peaks of the QRS complexes of an electrocardiogram
   ▪ Ventricular systoles
- Measuring the intervals between each subsequent two data points, preferably between successive two R-peaks of QRS complexes of an electrocardiogram.
- Calculating a quotient between an interval of interest, preferably an interval terminated by or initiated by a ventricular extrasystole, and a mean interval, wherein the mean interval is preferably calculated from at least one of the following:
   ▪ A number of consecutive intervals preceding and/or succeeding the interval of interest, wherein the number of consecutive intervals is preferably two, three, four, five, six, seven, eight, nine or ten
   ▪ A number of consecutive intervals preceding and/or succeeding a ventricular extrasystole, wherein the number of consecutive intervals is preferably two, three, four, five, six, seven, eight, nine or ten
- Selecting a data sequence for further processing in at least one of the following cases:
   ▪ The data sequence contains at least one data point correlating with a ventricular extrasystole
   ▪ The data sequence contains at least a number of consecutive data points correlating with regular ventricular systoles preceding and/or succeeding a ventricular extrasystole, preferably without interruption by any further ventricular extrasystole, wherein the number of consecutive data points correlating with regular ventricular systoles preceding and/or succeeding a ventricular extrasystole is preferably two, three, four, five, six, seven, eight, nine or ten
   ▪ The quotient calculated for at least one interval fulfills at least one mathematical criterion
   ▪ The quotients calculated for at least two subsequent intervals fulfill different mathematical criteria
   ▪ The quotients calculated for at least two subsequent intervals are out of a predetermined range of values
   ▪ The quotient calculated for a first interval of said data sequence is equal to or less than a first value, preferably 0.99, 0.97, 0.9 or 0.8 and/or the quotient calculated for a second interval subsequent to the first interval is equal to or greater than a second value, preferably 1.01, 1.03, 1.3 or 1.4
   ▪ The quotient calculated for an interval of said data sequence is equal to or less than a first value, preferably 0.99, 0.97, 0.9 or 0.8 and/or the quotient calculated for a number of subsequent consecutive intervals is equal to or greater than said first value, wherein the number of subsequent consecutive intervals is preferably two, three, four, five, six, seven, eight, nine or ten.

Still further preferably, said data processing unit is configured to compute a T-wave response to a ventricular extrasystole based on said data sequence, preferably by performing at least one of the following actions:
- Calculating the mean of T-wave amplitudes corresponding to a number of regular ventricular systoles preceding and/or succeeding the first post-extrasystolic T-wave amplitude, preferably without interruption by any further ventricular extrasystole, wherein the number of consecutive ventricular systoles preceding and/or succeeding said first post-extrasystolic T-wave amplitude is preferably two, three, four, five, six, seven, eight, nine or ten.
- Calculating the T-wave amplitude quotient between the first post-extrasystolic T-wave amplitude and the mean of T-wave amplitudes preceding and/or succeeding said first post-extrasystolic T-wave amplitude.
- Storing the calculated T-wave amplitude quotient or the mean of a plurality of calculated T-wave amplitude quotients under the variable PEST.
- Storing the number of calculated T-wave amplitudes, preferably per time.

According to a preferred embodiment of the invention, said data processing unit is configured to assess a mortality risk for said patient by providing at least one of the following functions:
- Allocating said patient to a low risk group under the following conditions:
   ▪ The number of data sequences selected per 30 minutes recording of said at least one data function is zero; or
   ▪ The number of data sequences selected per 30 minutes recording of said at least one data function is less than five; and
   ▪ The number of T-wave amplitude quotients calculated to be less than 1 per 30 minutes recording of said at least one data function is zero.
- Allocating said patient to a medium risk group under the following conditions:
   ▪ The number of data sequences selected per 30 minutes recording of said at least one data function is greater than five; and
   ▪ The number of T-wave amplitude quotients calculated to be less than 1 per 30 minutes recording of said at least one data function is zero.
- Allocating said patient to a high risk group under at least one of the following conditions:
   ▪ The number of data sequences selected per 30 minutes recording of said at least one data function is greater than five
   ▪ The number of T-wave amplitude quotients calculated to be less than 1 per 30 minutes recording of said at least one data function is greater than zero
- Assessing mortality risk for said patient by calculating a prognostic score based on at least one of the following:
   ▪ PEST
   ▪ VPC
   ▪ Heart Rate Turbulence, in particular HRT-TS ≤ 2.5 ms/RRI and/or HRT-TO ≥ 0 %

According to another preferred embodiment of the invention, said device comprises display means for displaying the result of mortality risk assessment.

The data provision unit and/or the data processing unit may be configured on an implantable device which is sized tobe implanted into a patients body.

Another preferred aspect of the invention relates to a method for assessing a mortality risk of a cardiac patient based on at least one vital sign or biosignal of said patient, preferably using the device according to at least one of the preceding claims, said method comprising the following steps:
- Providing at least one data function of at least one vital sign or biosignal of said patient.
- Processing data of said at least one data function for assessing a mortality risk of said patient by performing the following actions:
   ▪ Selecting at least one data sequence from said at least one data function according to a predetermined routine.
   ▪ Computing a specific behavior of at least one parameter of said at least one data function based on said at least one selected data sequence.
   ▪ Assessing a mortality risk of said patient based on said computed behavior.

Still another preferred aspect of the invention relates to a computer-readable medium containing a program, which, when loaded, performs a method for assessing a mortality risk of a cardiac patient based on at least one vital sign or biosignal of said patient comprising the following steps:
- Selecting at least one data sequence from at least one data function of at least one vital sign or biosignal of said patient according to a predetermined routine.
- Computing a specific behavior of at least one parameter of said at least one data function based on said at least one selected data sequence.
- Assessing a mortality risk of said patient based on said computed behavior.

The method preferably includes all features attributed to and provided by the device according to any one of the preceding embodiments.

Further preferred embodiments result from combinations of features disclosed in the subclaims with features disclosed in the specification and/or the drawings.

### Brief description of the drawings

- Fig. 1: shows exemplary plots of simultaneously recorded electrocardiogram and blood pressure curves of a cardiac patient having a low mortality risk assessment according to the teaching of the present invention, said plots showing data sequences including a ventricular extrasystole.
- Fig. 2: shows superimposed curves of data sequences indicating a systolic blood pressure response to a ventricular extrasystole of a plurality of cardiac patients having a low mortality risk assessment according to the teaching of the present invention, said data sequences including data points correlating with two cardiac contractions preceding said ventricular extrasystole and ten cardiac contractions succeeding said ventricular extrasystole, wherein the systolic blood pressure of the first post-extrasystolic cardiac contraction is lower than the mean systolic blood pressure of eight cardiac contractions subsequent to the first post-extrasystolic cardiac contraction.
- Fig. 3: shows exemplary plots of simultaneously recorded electrocardiogram and blood pressure curves of a cardiac patient having a high mortality risk assessment according to the teaching of the present invention, said plots showing data sequences including a ventricular extrasystole.
- Fig. 4: explains superimposed curves of data sequences indicating a systolic blood pressure response to a ventricular extrasystole of a plurality of cardiac patients having a high mortality risk assessment according to the teaching of the present invention, said data sequences including data points correlating with two cardiac contractions preceding said ventricular extrasystole and ten cardiac contractions succeeding said ventricular extrasystole, wherein the systolic blood pressure of the first post-extrasystolic cardiac contraction is greater than the mean systolic blood pressure of eight cardiac contractions subsequent to the first post-extrasystolic cardiac contraction.

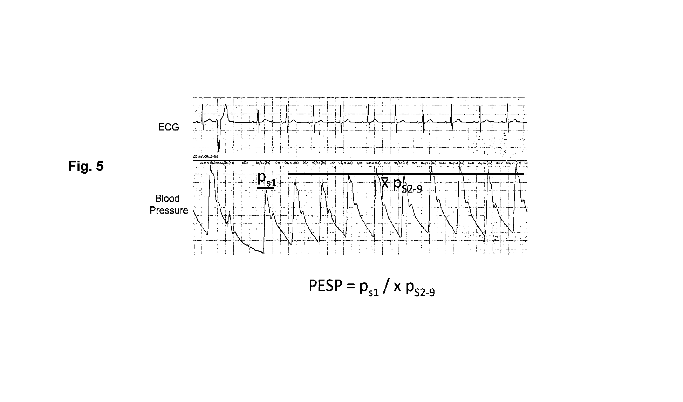

- Fig. 5: shows, on the basis of the exemplary plots according to Fig. 1, the routine according to the first embodiment of the invention for calculating a quotient between the systolic blood pressure of the first post-extrasystolic cardiac contraction and the mean systolic blood pressure of eight cardiac contractions subsequent to the first post-extrasystolic cardiac contraction.

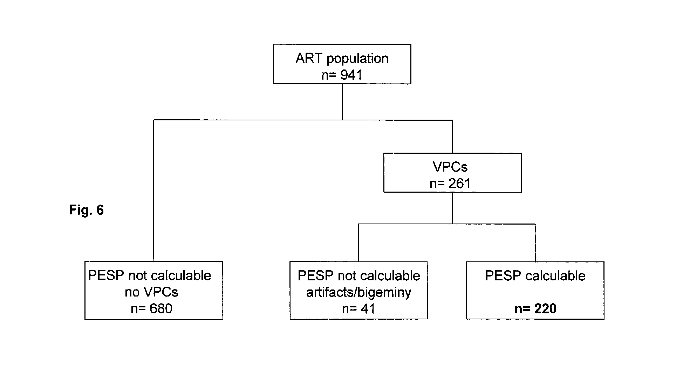

- Fig. 6: depicts the study population, wherein 680 out of 941 persons of the ART cohort did not show ventricular extrasystoles (VES) and PESP was not calcultable for 41 persons, so that the PESP cohort counts 220 persons.

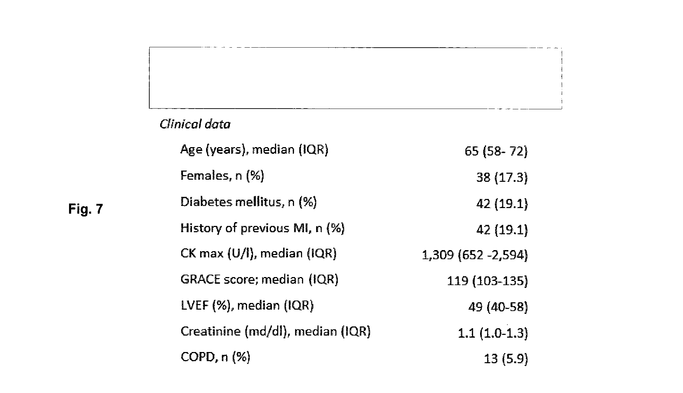

- Fig. 7: shows a table containing the clinical data of the PESP cohort.

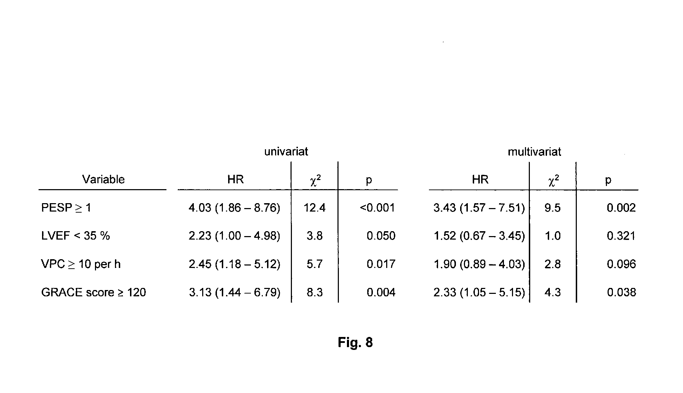

- Fig. 8: shows a table containing data calculated according to the Cox regression analysis and indicating the significance of PESP as a mortality risk predictor in relation to other known mortality risk predictors such as LVEF (Gold Standard), ventricular premature contractions (VPC) and the GRACE score.

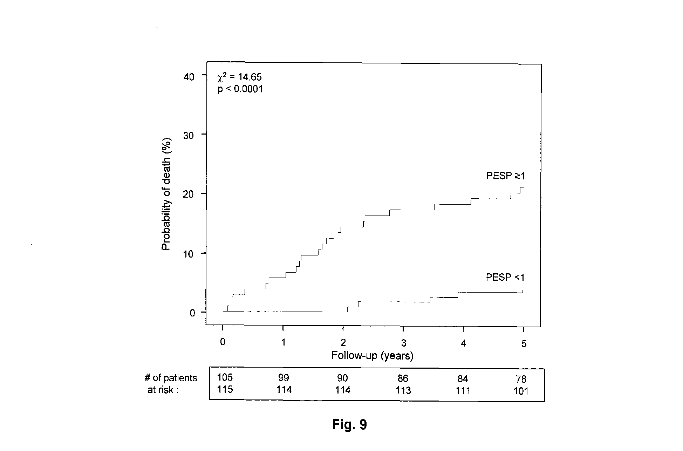

- Fig. 9: shows a survival curve estimated by the Kaplan-Meier method and indicating the mortality risk i.e. probability of death (%) over time following a myocardial infarction for myocardial infarction patients having a high mortality risk assessment according to the teaching of the first embodiment of the present invention (PESP >1) and for myocardial infarction patients having a low mortality risk assessment according to the teaching of the present invention (PESP <1).

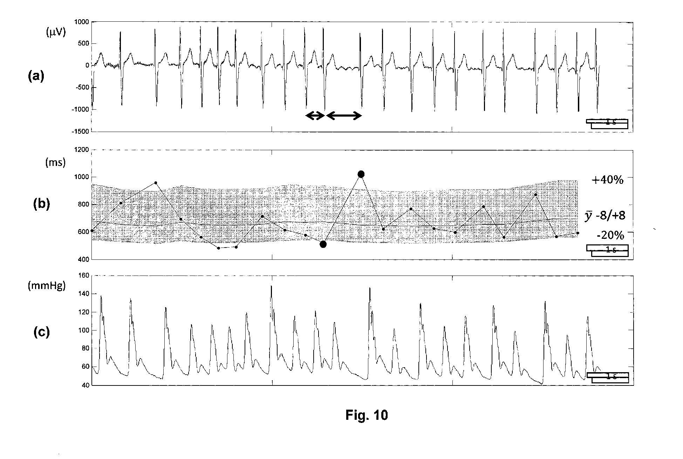

- Fig. 10: shows exemplary plots of simultaneous electrocardiogram (a), heart rate interval (b) and blood pressure curves (c) of a cardiac patient having a high mortality risk assessment according to the teaching of the second embodiment of the present invention, said plots showing data sequences during atrial fibrillation, wherein the data sequence contains two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

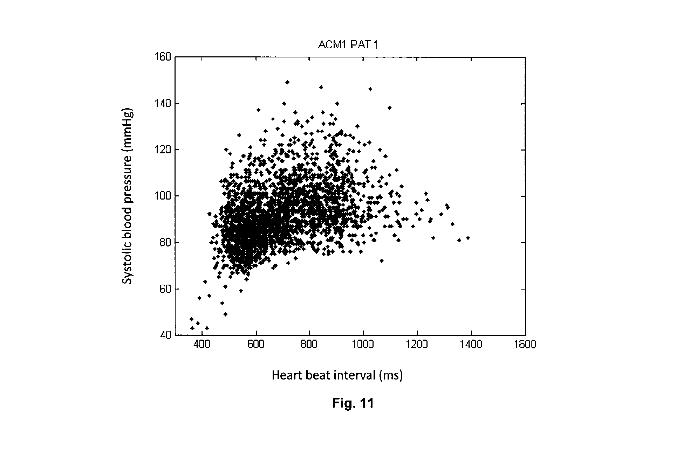

- Fig. 11: depicts a coordinate system with data points of systolic blood pressure (mmHg) over heart beat interval which are taken from data sequences of a cardiac patient having a high mortality risk assessment according to the teaching of the present invention, said data sequences containing two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

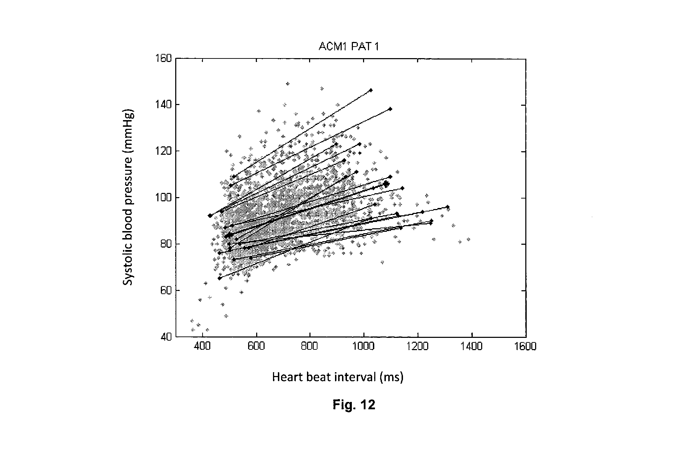

- Fig. 12: depicts the coordinate system of Fig. 11 with lines connecting respective two data points correlating with the two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

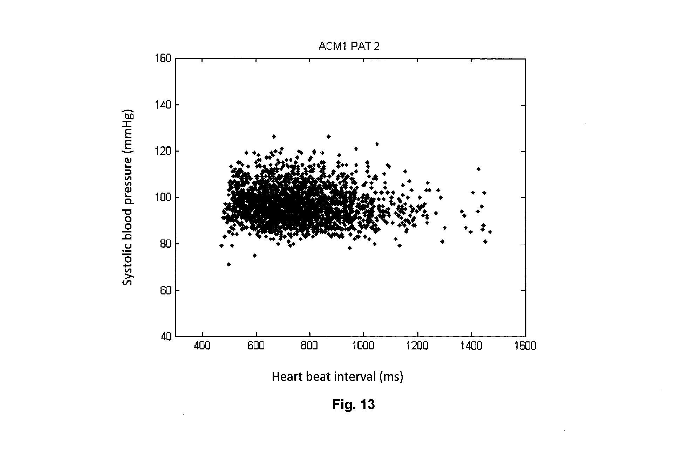

- Fig. 13: depicts a coordinate system with data points of systolic blood pressure (mmHg) over heart beat interval which are taken from data sequences of cardiac patients having a low mortality risk assessment according to the teaching of the second embodiment of the present invention, said data sequences containing two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

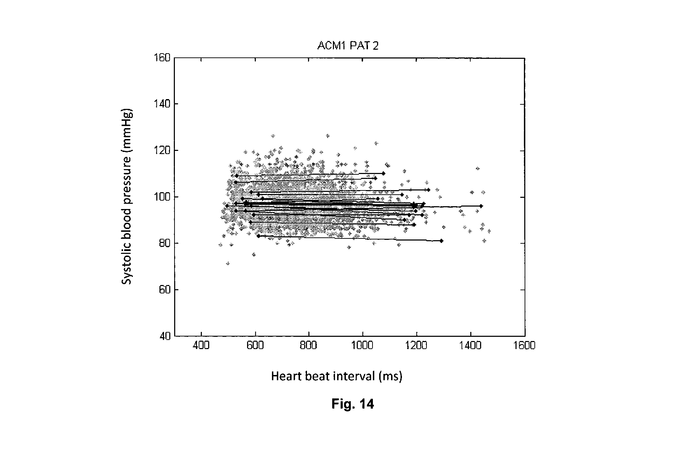

- Fig. 14: depicts the coordinate system of Fig. 11 with lines connecting respective two data points correlating with the two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

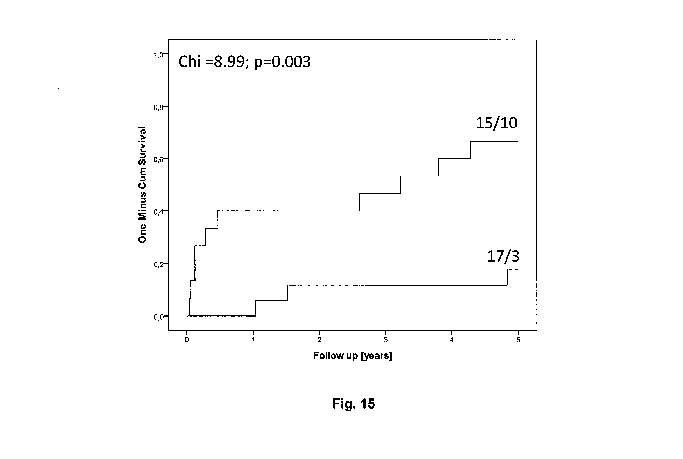

- Fig. 15: shows a survival curve estimated by the Kaplan-Meier method and indicating the mortality risk i.e. probability of death (%) over time following a myocardial infarction for myocardial infarction patients having a high mortality risk assessment according to the teaching of the second embodiment of the present invention (15/10) and for myocardial infarction patients having a low mortality risk assessment according to the teaching of the present invention (17/3).

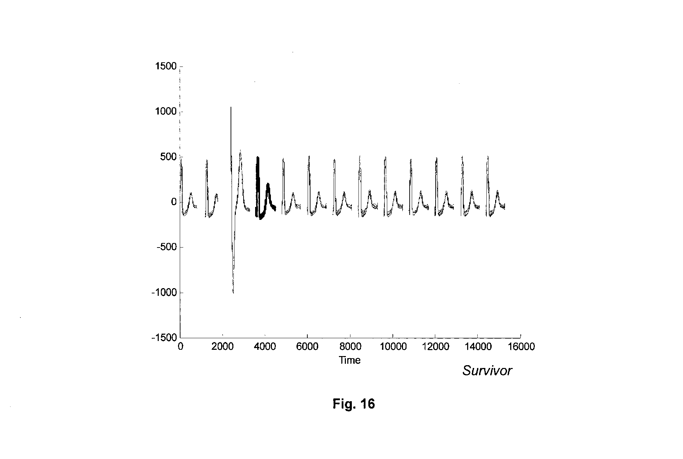

- Fig. 16: depicts an electrocardiogram recording over the time of a cardiac patient having a low mortality risk assessment according to the teaching of the third embodiment of the present invention, wherein the first post-extrasystolic T-wave amplitude is greater than the mean of three T-wave amplitudes preceding the ventricular extrasystole and three T-wave amplitudes succeeding the ventricular extrasystole.

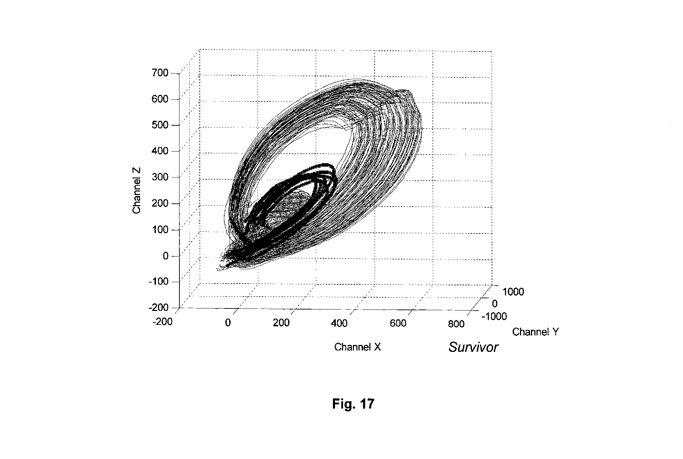

- Fig. 17: depicts the data of the electrocardiogram recording of Fig. 16 in a three-dimensional coordinate system.

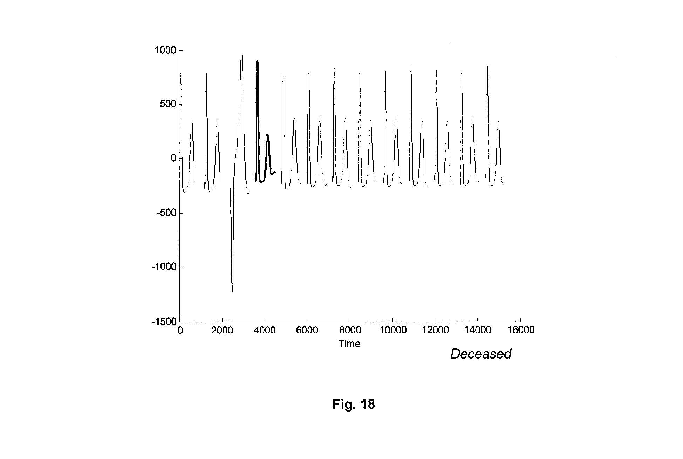

- Fig. 18: depicts an electrocardiogram recording over the time of a cardiac patient having a high mortality risk assessment according to the teaching of the third embodiment of the present invention, wherein the first post-extrasystolic T-wave amplitude is lower than the mean of three T-wave amplitudes preceding the ventricular extrasystole and three T-wave amplitudes succeeding the ventricular extrasystole.

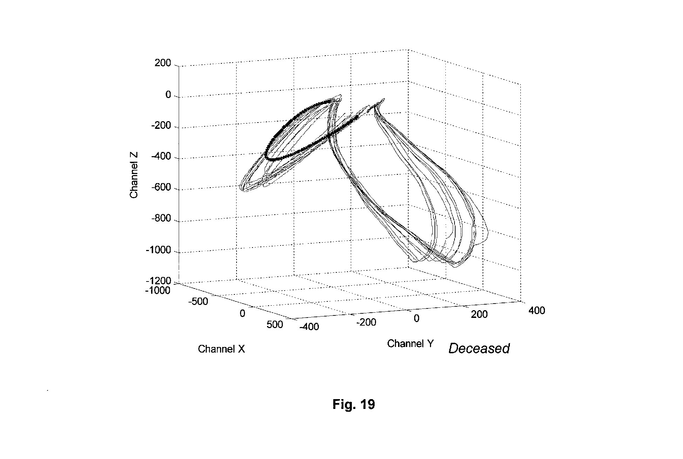

- Fig. 19: depicts the data of the electrocardiogram recording of Fig. 18 in a three-dimensional coordinate system.

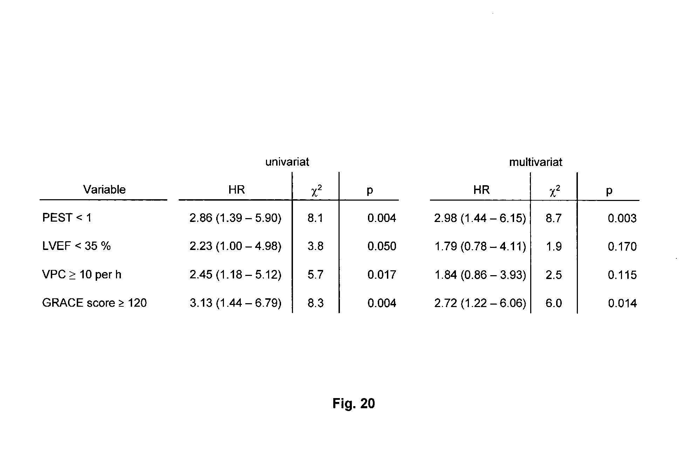

- Fig. 20: shows a table containing data calculated according to the Cox regression analysis and indicating the significance of PEST as a mortality risk predictor in relation to other known mortality risk predictors such as LVEF (Gold Standard), ventricular premature contractions (VPC) and the GRACE score.

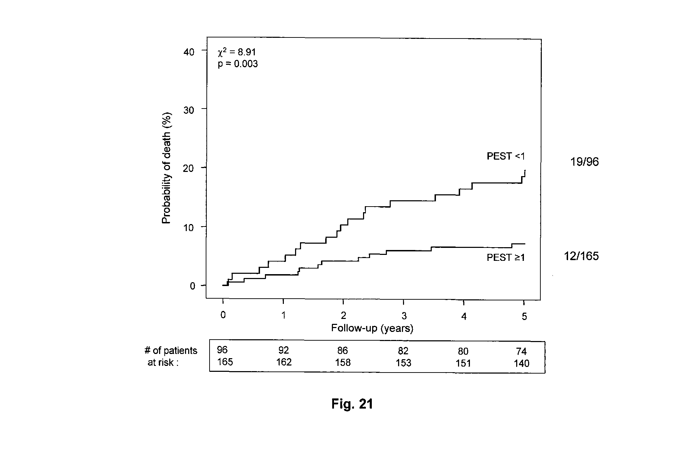

- Fig. 21: contains a diagram indicating the mortality risk i.e. probability of death (%) over the years following a myocardial infarction for myocardial infarction patients having a high mortality risk assessment according to the teaching of the third embodiment of the present invention (PEST < 1) and for myocardial infarction patients having a low mortality risk assessment according to the teaching of the third embodiment of the present invention (PEST ≥ 1).

### Detailed description of the preferred embodiments:

### Data provision unit

The data provision unit is configured to provide simultaneous 30-minute recordings of high resolution ECG of the patient within two weeks after myocardial infarction, and non-invasive arterial blood pressure monitoring of the patient using a finger photoplethysmographic device. Recordings should be made in supine resting position after routine morning medications and the raw signals should be verified including the elimination of artefacts where needed, wherein QRS classifications should be carefully reviewed and manually corrected as appropriate to differentiate sinus and ventricular premature complexes (VPC) or ventricular extrasystoles (VES). The data my be recorded and sotred in digital form.

### Data processing unit

The data processing unit is embodied as analysis software in the preferred embodiments of the invention.

**First example (****Figs. 1 to 9****) - Post-Extrasystolic Potentiation (PESP)**

According to an example PESP will be used as the mortality risk predictor. In the first example simultaneous recordings of electrocardiogram (ECG) and blood pressure of a cardiac patient provided by the data provision unit will be analyzed by the data processing unit by performing the following steps:
1) Recognition of QRS-complexes from ECG recordings, including identification of ventricular extrasystoles (VES) and determination of heart rate intervals (RRI) (cf. Figs. 1 and 3).
2) Selection of data sequences including VES, the coupling intervals of which (i.e. the intervals terminated by the VES) being shortened by at least x%, preferably 20%, and the post-extrasystolic pauses or breaks (i.e. the intervals initiated by the VES) being at least y%, preferably 40%, longer than the mean of z intervals, preferably five intervals, prior to the ventricular extrasystole (VES) (cf. Figs. 1 and 3).
3) Detection of the systolic arterial blood pressure of the first ten regular heart beats subsequent to the ventricular extrasystole (VES) (cf. Figs. 2 and 4).
4) Calculation of a PESP1 quotient between the systolic blood pressure of the first post-extrasystolic heart beat and the mean of the systolic blood pressure of the subsequent eight (PESP1 = P_{S1}/*x̅*P_{S2}-_{S9}) or nine (PESP1 = P_{S1}/*x̅*P_{S2}-_{S10}) heart beats (cf. Fig. 5).
5) assessing mortality risks of said patient based on the computed PESP1 quotient, wherein PESP quotients of >1 are found to be pathological.

A technical filter could possibly be included, e.g., by 3% in order to minimize the effect of basic noise.

Alternatively to no. 2, a data sequence containing a ventricular extrasystole (VES), i.e. a VPC, may be selected for calculation of the PESP1 quotient if the VES coupling interval is ≥ 20% premature compared to the mean of preceding five intervals, and if followed by ≥ 10 consecutive sinus beats. Post-ectopic sequences interrupted by artefacts or further ectopic beats should not be used.

The data processing unit is further configured to store the number of detected VPC and the number of PESP1 quotients greater than 1, and to average the calculated PESP1 quotients if the number of calculated PESP1 quotients within the 30 minute recording is greater than one. VPC count is defined as the number of VPCs within the 30 minute recording (irrespective of whether the VPCs qualified for PESP assessment).

The mortality risk for said cardiac patient can be assessed on the basis of the number of detected VPCs and PESP1 quotient.

A study with a study cohort according to Figs. 6 and 7 aimed at validating the predictive value of PESP in conjunction with three standard risk predictors, namely VPC count, GRACE score and LVEF.

VPC is defined as the number of VPCs within the 30 minute recording (irrespective of whether the VPCs qualified for PESP assessment).

GRACE score is calculated according to Eagle et al. The elements of the GRACE score included age, history of past heart failure, history of myocardial infarction, serum creatinine at admission, cardiac biomarkers status at admission, SBP at admission, pulse at admission, ST deviation at admission, and in-hospital percutaneous coronary intervention.

LVEF is assessed by left ventricular angiography or biplane echocardiography (Sonos 5500, Hewlett Packard, Palo Alto, CA, USA).

GRACE score and LVEF were dichotomized at predefined cutoff values of ≥120 and ≤35%, respectively. VPC count was dichotomized at its median of ≥5 per 30 minutes.

A Cox proportional hazards model was used with all variables entered simultaneously to assess the independence and prognostic value of mortality predictors. Survival curves were estimated by the Kaplan-Meier method and compared by the log-rank test (Fig. 9). The correlation between the four prognostic markers was analysed by the chi-square test (Fig. 8). Differences were considered statistically significant if p < 0.05 (IBM SPSS Statistics 20.0, SPSS Inc.).

### Second example (Figs. 10 to 15) - PESP in case of atrial fibrillation

Same as the first example, the second example of the invention uses PESP as mortality risk predictor. However, the second example applies to cardiacs patient suffering from atrial fibrillation which hinders the detection of ventricular extrasystole (VES) from the electrocardiogram (ECG). Again, simultaneous recordings of electrocardiogram (ECG) and blood pressure of a cardiac patient provided by the data provision unit will be analyzed by the data processing unit, wherein the data processing unit performs the following steps:
1) Recognition of QRS-complexes, determination of heart rate intervals (RRI).
2) Calculation of a quotient between each RRI and the mean of 8 preceding and succeeding RRIs (RRIᵢ/*x̅*RRI_{i-8..i+8}).
3) Identification of consecutive two RRIs, which meet the following criteria: RRIᵢ < 80% *x̅*RRI_{i-8..i+8}; RRIᵢ₊₁ >140% *x̅*RRI_{i+1-8..i+1+8}
4) Identification of the systolic blood pressure of the heart beat terminating RRIᵢ und RRIᵢ₊₁.
5) Under the presumption of linear relation between RRI and systolic blood pressure (Psys), calculation of PESP2 as the inclination of the linear function defined by the two data points (RRIᵢ | Psysᵢ) and (RRIᵢ₊₁ | Psysᵢ₊₁).
6) In case of several two consecutive intervals meeting the criteria under 3), averaging the PESP2 values calculated for the separate double interval sequences.
7) Assessing a mortality risk based on the calculated PESP2, wherein PESP2 > 4.5 mmHg/s counts as pathological.

As shown in Fig. 10 (a) and (b), the depicted data sequence of the electrocardiogram (ECG) contains two consecutive heart rate intervals, for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

Fig. 11 shows a coordinate system of systolic blood pressure (mmHg) over heart beat interval including data points taken e.g. from the data sequences shown in Fig. 10 (b) and (c). These data sequences originate from myocardial infarction patients having a high mortality risk assessment according to the teaching of the second example and contain two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4.

When connecting two corresponding data points of Fig. 11 with lines, the coordinate system according to Fig. 12 results. PESP2 corresponds to the inclination of a line, or the averaged inclination of a plurality of lines connecting two corresponding data points of two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4. PESP2 of greater than 4.5 mmHg/s is regarded as pathologic.

Figs. 13 and 14 depict coordinate systems similar to Figs. 11 and 12 but include data points taken from the data sequences which originate from cardiac patients having a low mortality risk assessment according to the teaching of the second example. As can be seen in Fig. 14, the lines connecting two corresponding data points of two consecutive heart rate intervals for which the quotient between the first heart rate interval to the mean of eight preceding and eight succeeding heart rate intervals is less than 0.8 and the quotient between the second heart rate interval to the mean of eight preceding and eight succeeding heart rate intervals is greater than 1.4, are almost parallel to the abscissa. The PESP2 variable calculated from the data points depicted in Fig. 14 amount to about 0.0 mmHg/s and can be regarded as an indicator of a low mortality risk.

### The Invention (Figs. 15 to 21) - Post-Extrasystolic T-wave change (PEST)

Unlike the first and second example the invention uses PEST, i.e. a quotient indicative of Post-Extrasystolic T-wave augmentation as mortality risk predictor. According to the invention only recordings of electrocardiogram (ECG) of a cardiac patient provided by the data provision unit will be analyzed by the data processing unit by performing the following steps:
1) Recognition of QRS-complexes, identification of VES.
2) Selection of data sequences including VES, wherein each data sequence comprises three regular QRS-complexes preceding the VES and three QRS-complexes succeeding the VES.
3) Quantification of T-wave amplitudes of the selected six heart beats through planimetry (F_{T-3,-2,-1,-1,+2,-3}).
4) Calculation of PEST as the quotient of the first post-extrasystolic T-wave amplitude (F_{T}) to the mean of the remaining five T-wave amplitudes (*x̅*F_{T-3,-2,-1,+2,+3}) of the selected data sequence according to the formula: PEST = F_{T+1} / *x̅*F_{T}-3,-2,-i.+2,+3
5) Assessing the mortality risk of said patient based on PEST, wherein PEST <1 counts as pathological.

Again, a Cox proportional hazards model was used with all variables entered simultaneously to assess the independence and prognostic value of mortality predictors. Survival curves were estimated by the Kaplan-Meier method and compared by the log-rank test (Fig. 20). The correlation between the four prognostic markers was analysed by the chi-square test (Fig. 21). Clinical post-infarction studies revealed that PESP and PEST calculated according to the present invention are strong and independent predictors of mortality risk of myocardial infarction patient.

A prognostic score based on PESP, PEST, VPC and Heart Rate Turbulence (HRT-TS ≤ 2.5 ms/RRI and/or HRT-TO ≥ 0 %) provides superior results in mortality risk stratification as compared to conventional mortality predictors such as GRACE and LVEF.

## Claims

1. Device for assessing a mortality risk of a cardiac patient based on at least one vital sign or biosignal of said patient, said device comprising:
a. A data provision unit being configured to provide at least one recording of electrocardiogram (ECG) as at least one data function of said at least one vital sign or biosignal of said patient.
b. A data processing unit being configured to process data of said at least one data function for assessing a mortality risk of said patient by performing the following actions:
i. Selecting at least one data sequence from said at least one data function according to a predetermined routine, wherein the data sequence contains at least one data point correlating with a ventricular extrasystole, said device being **characterized by:**
ii. Computing a specific T-wave response to a ventricular extrasystole based on said at least one selected data sequence by calculating the T-wave amplitude quotient between the first post-extrasystolic T-wave amplitude and the mean of T-wave amplitudes preceding and/or succeeding said first post-extrasystolic T-wave amplitude.
iii. Assessing a mortality risk of said patient based on said computed T-wave response to a ventricular extrasystole by allocating said patient to a high risk group in case the number of T-wave amplitude quotients calculated to be less than 1 per 30 minutes recording of said data function is greater than zero.

2. Device according to claim 1, **characterized by** said data provision unit being configured to perform at least one of the following actions:
a. Recording said at least one vital sign or biosignal, preferably in digital form.
b. Recording said at least one vital sign or biosignal continuously for a predetermined period of time, preferably for at least 30 minutes.
c. Recording said at least one vital sign or biosignal with non-invasive recording means, preferably using the following device:
i. An electrocardiogram recorder, preferably a high resolution electrocardiogram recorder with at least 1.6 kHz in orthogonal XYZ leads, preferably for continuously recording an electrocardiogram of said patient.
d. Recording said at least one vital sign or biosignal in a resting position of said patient, preferably in a supine resting position of said patient.
e. Recording at least two different vital signs or biosignals simultaneously, preferably an electrocardiogram and blood pressure, preferably continuous arterial blood pressure.
f. Storing the recorded data in data storage means, preferably in digital form.
g. Providing said at least one data function as a function of time.
h. Enabling verification and/or review and/or manual correction of said at least one data function, in particular by enabling review and/or manual correction of QRS classifications so as to differentiate sinus and ventricular premature complexes (VPC).
i. Loading said at least one data function from data storage means, preferably in digital form.

3. Device according to one of the preceding claims 1 to 2, **characterized by** said data processing unit being configured to select said at least one data sequence from said at least one data function by performing at least one of the following actions:
a. Identifying periodic patterns within said at least one data function, preferably QRS-complexes.
b. Identifying data points of said at least one data function correlating with:
i. R-peaks of the QRS complexes of an electrocardiogram
c. Measuring the intervals between each subsequent two data points, preferably between successive two R-peaks of QRS complexes.
d. Calculating a quotient between an interval of interest and a mean interval, wherein the mean interval can be calculated from at least one of the following:
i. A number of consecutive intervals preceding and/or succeeding the interval of interest, wherein the number of consecutive intervals is preferably two, three, four, five, six, seven, eight, nine or ten
ii. A number of consecutive intervals preceding and/or succeeding a ventricular extrasystole, wherein the number of consecutive intervals is preferably two, three, four, five, six, seven, eight, nine or ten
e. Selecting a data sequence for further processing in at least one of the following cases:
i. The data sequence contains at least a number of consecutive data points correlating with regular ventricular systoles preceding and/or succeeding a ventricular extrasystole
ii. The quotient calculated for at least one interval fulfills at least one mathematical criterion
iii. The quotients calculated for at least two subsequent intervals fulfill different mathematical criteria
iv. The quotients calculated for at least two subsequent intervals are out of a predetermined range of values
v. The quotient calculated for a first interval of said data sequence is equal to or less than a first value and/or the quotient calculated for a second interval subsequent to the first interval is equal to or greater than a second value
vi. The quotient calculated for an interval of said data sequence is equal to or less than a first value and/or the quotient calculated for a number of subsequent consecutive intervals is equal to or greater than said first value.

4. Device according to one of the preceding claims 1 to 3, **characterized by** said data processing unit being configured to compute a T-wave response to a ventricular extrasystole based on said data sequence by performing at least one of the following actions:
a. Storing the calculated T-wave amplitude quotient or the mean of a plurality of calculated T-wave amplitude quotients under the variable PEST.
b. Storing the number of calculated T-wave amplitudes, preferably per time.

5. Device according to one of the preceding claims 1 to 4, **characterized by** said data processing unit being configured to assess a mortality risk for said patient by providing at least one of the following functions:
a. Allocating said patient to a low risk group under the following conditions:
i. The number of data sequences selected per 30 minutes recording of said at least one data function is less than five; and
ii. The number of T-wave amplitude quotients calculated to be less than 1 per 30 minutes recording of said at least one data function is zero.
b. Allocating said patient to a medium risk group under the following conditions:
i. The number of data sequences selected per 30 minutes recording of said at least one data function is greater than five; and
ii. The number of T-wave amplitude quotients calculated to be less than 1 per 30 minutes recording of said at least one data function is zero.
c. Allocating said patient to a high risk group under at least one of the following conditions:
i. The number of data sequences selected per 30 minutes recording of said at least one data function is greater than five
ii. PEST is less than one
d. Assessing mortality risk for said patient by calculating a prognostic score based on at least one of the following:
i. PEST
ii. VPC
iii. Heart Rate Turbulence, in particular HRT-TS ≤ 2.5 ms/RRI and/or HRT-TO ≥ 0 %

6. Device according to one of the preceding claims 1 to 5, **characterized by** said device comprising display means for displaying the result of mortality risk assessment.

7. Method for assessing a mortality risk of a cardiac patient based on at least one vital sign or biosignal of said patient, said method comprising the following steps:
a. Providing by a data provision unit at least one recording of electrocardiogram (ECG) as at least one data function of said at least one vital sign or biosignal of said patient.
b. Processing by a data processing unit data of said at least one data function for assessing a mortality risk of said patient by performing the following actions:
i. Selecting at least one data sequence from said at least one data function according to a predetermined routine, wherein the data sequence contains at least one data point correlating with a ventricular extrasystole said method being **characterized by:**
ii. Computing a specific T-wave response to a ventricular extrasystole based on said at least one selected data sequence by calculating the T-wave amplitude quotient between the first post-extrasystolic T-wave amplitude and the mean of T-wave amplitudes preceding and/or succeeding said first post-extrasystolic T-wave amplitude.
iii. Assessing a mortality risk of said patient based on said computed T-wave response by allocating said patient to a high risk group in case the number of T-wave amplitude quotients calculated to be less than 1 per 30 minutes recording of said data function is greater than zero.

8. Computer-readable medium containing a program, which, when loaded, performs a method for assessing a mortality risk of a cardiac patient based on at least one vital sign or biosignal of said patient comprising the following steps:
a. Selecting, according to a predetermined routine, at least one data sequence from at least one recording of electrocardiogram (ECG) which is provided as at least one data function of said at least one vital sign or biosignal of said patient, wherein the data sequence contains at least one data point correlating with a ventricular extrasystole, said program contained on said computer-readable medium being **characterized by:**
b. Computing a specific T-wave response to a ventricular extrasystole based on said at least one selected data sequence by calculating the T-wave amplitude quotient between the first post-extrasystolic T-wave amplitude and the mean of T-wave amplitudes preceding and/or succeeding said first post-extrasystolic T-wave amplitude.
c. Assessing a mortality risk of said patient based on said computed T-wave response by allocating said patient to a high risk group in case the number of T-wave amplitude quotients calculated to be less than 1 per 30 minutes recording of said data function is greater than zero.

## Patentansprüche

1. Vorrichtung zum Bewerten eines Sterblichkeitsrisikos eines Herzpatienten auf der Grundlage von wenigstens einem Lebenszeichen oder Biosignal des Patienten, wobei die Vorrichtung aufweist:
a. eine Einheit zum Bereitstellen von Daten, die so konfiguriert ist, dass sie wenigstens eine Aufzeichnung eines Elektrokardiogramms (EKG) als wenigstens eine Datenfunktion des wenigstens einen Lebenszeichens oder Biosignals des Patienten bereitstellt.
b. eine Datenverarbeitungseinheit, die so konfiguriert ist, dass sie Daten der wenigstens einen Datenfunktion zum Bewerten eines Sterblichkeitsrisikos des Patienten verarbeitet, indem sie die folgenden Schritte durchführt:
i. Auswählen von wenigstens einer Datensequenz aus der wenigstens einen Datenfunktion gemäß einer vorab festgelegten Routine, wobei die Datensequenz wenigstens einen Datenpunkt enthält, der mit einer ventrikulären Extrasystole korreliert, wobei die Vorrichtung **gekennzeichnet ist durch:**
ii. Berechnen einer spezifischen T-Wellen-Antwort auf eine ventrikuläre Extrasystole auf der Grundlage der wenigstens einen ausgewählten Datensequenz **durch** Berechnen des Quotienten der T-Wellen-Amplitude zwischen der ersten post-extrasystolischen T-Wellen-Amplitude und dem Mittel aus T-Wellen-Amplituden, die der ersten post-extrasystolischen T-Wellen-Amplitude vorausgehen und/oder folgen.
iii. Bewerten eines Sterblichkeitsrisikos des Patienten auf der Grundlage der berechneten T-Wellen-Antwort auf eine ventrikuläre Extrasystole **durch** Zuweisen des Patienten zu einer Gruppe mit hohem Risiko, wenn die Anzahl von Quotienten aus T-Wellen-Amplituden, die laut Berechnung kleiner als 1 pro 30 Minuten Aufzeichnung der Datenfunktion ist, größer als Null ist.

2. Vorrichtung nach Anspruch 1, **dadurch gekennzeichnet, dass** die Einheit zum Vorsehen von Daten so konfiguriert ist, dass sie wenigstens einen der folgenden Schritte durchführt:
a. Aufzeichnen des wenigstens einen Lebenszeichens oder Biosignals, vorzugsweise in digitaler Form.
b. Aufzeichnen des wenigstens einen Lebenszeichens oder Biosignals durchgehend über einen vorab festgelegten Zeitraum, vorzugsweise für wenigstens 30 Minuten.
c. Aufzeichnen des wenigstens einen Lebenszeichens oder Biosignals mit einer nicht-invasiven Aufzeichnungsvorrichtung, vorzugsweise unter Verwendung der folgenden Vorrichtung:
i. Eine Elektrokardiogramm-Aufzeichnungsvorrichtung, vorzugsweise eine Elektrokardiogramm-Aufzeichnungsvorrichtung mit einer hohen Auflösung mit wenigstens 1,6 kHz in orthogonalen XYZ-Kabeln, vorzugsweise zum durchgehenden Aufzeichnen eines Elektrokardiogramms des Patienten.
d. Aufzeichnen des wenigstens einen Lebenszeichens oder Biosignals in einer ruhenden Position des Patienten, vorzugsweise in einer auf dem Rücken liegenden ruhenden Position des Patienten.
e. Gleichzeitiges Aufzeichnen von wenigstens zwei unterschiedlichen Lebenszeichen oder Biosignalen, vorzugsweise eines Elektrokardiogramms und eines Blutdruckes, vorzugsweise eines kontinuierlichen arteriellen Blutdruckes.
f. Speichern der aufgezeichneten Daten in einer Datenspeichervorrichtung, vorzugsweise in digitaler Form.
g. Vorsehen der wenigstens einen Datenfunktion als eine Funktion der Zeit.
h. Aktivieren einer Verifizierung und/oder Überprüfung und/oder manuellen Korrektur der wenigstens einen Datenfunktion, insbesondere durch Aktivieren von Überprüfung und/oder manueller Korrektur von QRS-Klassifizierungen, um zwischen Sinus- und ventrikulären vorzeitigen Komplexen (VPC) zu unterscheiden.
i. Laden der wenigstens einen Datenfunktion aus der Datenspeichervorrichtung, vorzugsweise in digitaler Form.

3. Vorrichtung nach einem der vorstehenden Ansprüche 1 oder 2, **dadurch gekennzeichnet, dass** die Datenverarbeitungseinheit so konfiguriert ist, dass sie wenigstens eine Datensequenz aus der wenigstens einen Datenfunktion durch Durchführen wenigstens eines der folgenden Schritte auswählt:
a. Identifizieren von periodischen Mustern innerhalb der wenigstens einen Datenfunktion, vorzugsweise von QRS-Komplexen.
b. Identifizieren von Datenpunkten der wenigstens einen Datenfunktion, die korrelieren mit:
i. R-Spitzen der QRS-Komplexe eines Elektrokardiogramms
c. Messen der Intervalle zwischen jeden zwei aufeinanderfolgenden Datenpunkten, vorzugsweise zwischen zwei aufeinanderfolgenden R-Spitzen von QRS-Komplexen.
d. Berechnen eines Quotienten zwischen einem Intervall von Interesse und einem mittleren Intervall, wobei das mittlere Intervall anhand von wenigstens einem der Folgenden berechnet werden kann:
i. eine Anzahl von aufeinanderfolgenden Intervallen, die dem Intervall von Interesse vorausgehen und/oder folgen, wobei die Anzahl von aufeinanderfolgenden Intervallen vorzugsweise zwei, drei, vier, fünf, sechs, sieben, acht, neun oder zehn beträgt.
ii. eine Anzahl von aufeinanderfolgenden Intervallen, die einer ventrikulären Extrasystole vorausgehen und/oder folgen, wobei die Anzahl von aufeinanderfolgenden Intervallen zwei, drei, vier, fünf, sechs, sieben, acht, neun oder zehn beträgt.
e. Auswählen von wenigstens einer Datensequenz zur weiteren Verarbeitung in mindestens einem der folgenden Fälle:
i. Die Datensequenz enthält mindestens eine Anzahl aufeinanderfolgender Datenpunkte, die mit regulären ventrikulären Extrasystolen korrelieren, die einer ventrikulären Extrasystole vorausgehen und/oder ihr folgen.
ii. Der für wenigstens ein Intervall berechnete Quotient erfüllt wenigstens ein mathematisches Kriterium.
iii. Die für wenigstens zwei aufeinanderfolgende Intervalle berechneten Quotienten erfüllen unterschiedliche mathematische Kriterien.
iv. Die für wenigstens zwei aufeinanderfolgende Intervalle berechneten Quotienten liegen außerhalb eines vorab festgelegten Wertebereiches.
v. Der für ein erstes Intervall der Datensequenz berechnete Quotient ist gleich oder kleiner als ein erster Wert und/oder der Quotient, der für ein zweites, sich an das erste Intervall anschließende Intervall berechnet wird, ist gleich oder größer als ein zweiter Wert.
vi. Der für ein Intervall der Datensequenz berechnete Quotient ist gleich oder kleiner als ein erster Wert und/oder der Quotient, der für eine Anzahl von sich anschließenden aufeinanderfolgenden Intervallen berechnet wird, ist gleich oder größer als der erste Wert.

4. Vorrichtung nach einem der vorstehenden Ansprüche 1 bis 3, **dadurch gekennzeichnet, dass** die Datenverarbeitungseinheit so konfiguriert ist, dass sie eine T-Wellen-Antwort auf eine ventrikuläre Extrasystole auf der Grundlage der Datensequenz berechnet, indem sie wenigstens einen der folgenden Schritte durchführt:
a. Speichern des berechneten Quotienten der T-Wellen-Amplituden oder des Mittels einer Vielzahl von berechneten Quotienten von T-Wellen-Amplituden in der Variable PEST.
b. Speichern der Anzahl von berechneten T-Wellen-Amplituden, vorzugsweise pro Zeit.

5. Vorrichtung nach einem der vorstehenden Ansprüche 1 bis 4, **dadurch gekennzeichnet, dass** die Datenverarbeitungseinheit so konfiguriert ist, dass sie ein Sterblichkeitsrisiko für den Patienten durch Bereitstellen von wenigstens einer der folgenden Funktionen bewertet:
a. Zuweisen des Patienten zu einer Gruppe geringen Risikos unter den folgenden Bedingungen:
i. Die Anzahl von Datensequenzen, die pro 30 Minuten Aufzeichnung der wenigstens einen Datenfunktion ausgewählt wurde, beträgt weniger als fünf; und
ii. Die Anzahl von Quotienten aus T-Wellen-Amplituden, die laut Berechnung kleiner als 1 pro 30 Minuten Aufzeichnung der wenigstens einen Datenfunktion beträgt, beträgt Null.
b. Zuweisen des Patienten zu einer Gruppe mittleren Risikos unter den folgenden Bedingungen:
i. Die Anzahl von Datensequenzen, die pro 30 Minuten Aufzeichnung der wenigstens einen Datenfunktion ausgewählt wurde, ist größer als fünf; und
ii. Die Anzahl von Quotienten aus T-Wellen-Amplituden, die laut Berechnung weniger als 1 pro 30 Minuten Aufzeichnung der wenigstens einen Datenfunktion beträgt, beträgt Null.
c. Zuweisen des Patienten zu einer Gruppe mit hohem Risiko unter wenigstens einer der folgenden Bedingungen:
i. Die Anzahl von Datensequenzen, die pro 30 Minuten Aufzeichnung der wenigstens einen Datenfunktion ausgewählt wurde, ist größer als fünf;
ii. PEST ist kleiner als 1.
d. Bewerten des Sterblichkeitsrisikos für den Patienten durch Berechnen einer prognostischen Wertung auf der Grundlage von wenigstens einem der folgenden:
i. PEST
ii. VPC
iii. Herzfrequenz-Turbulenz, insbesondere HRT-TS ≤ 2,5 ms/RRI und/oder HRT-TO ≥ 0%

6. Vorrichtung nach einem der vorstehenden Ansprüche 1 bis 5, **dadurch gekennzeichnet, dass** die Vorrichtung eine Anzeigevorrichtung zum Anzeigen des Ergebnisses der Bewertung des Sterblichkeitsrisikos umfasst.

7. Verfahren zum Bewerten eines Sterblichkeitsrisikos eines Herzpatienten auf der Grundlage von wenigstens einem Lebenszeichen oder Biosignal des Patienten, wobei das Verfahren die folgenden Schritte umfasst:
a. Vorsehen [...], durch eine Einheit zum Vorsehen von Daten [...], von wenigstens einer Aufzeichnung eines Elektrokardiogramms (EKG) als wenigstens eine Datenfunktion des wenigstens einen Lebenszeichens oder Biosignals des Patienten.
b. Verarbeiten [...], durch eine Datenverarbeitungseinheit [...], von Daten der wenigstens einen Datenfunktion für das Bewerten eines Sterblichkeitsrisikos des Patienten, indem die folgenden Schritte durchgeführt werden:
i. Auswählen von wenigstens einer Datensequenz aus der wenigstens einen Datenfunktion gemäß einer vorab festgelegten Routine, wobei die Datensequenz wenigstens einen Datenpunkt enthält, der mit einer ventrikulären Extrasystole korreliert, , wobei das Verfahren **gekennzeichnet ist durch:**
ii. Berechnen einer spezifischen T-Wellen-Antwort auf eine ventrikuläre Extrasystole auf der Grundlage von wenigstens einer ausgewählten Datensequenz **durch** Berechnen des Quotienten der T-Wellen-Amplituden zwischen der ersten post-extrasystolischen T-Wellen-Amplitude und dem Mittel aus T-Wellen-Amplituden, die der ersten post-extrasystolischen T-Wellen-Amplitude vorausgehen und/oder folgen.
iii. Bewerten eines Sterblichkeitsrisikos des Patienten auf der Grundlage der berechneten T-Wellen-Antwort **durch** Zuweisen des Patienten zu einer Gruppe mit hohem Risiko, wenn die Anzahl von Quotienten aus T-Wellen-Amplituden, die laut Berechnung kleiner als 1 pro 30 Minuten Aufzeichnung der Datenfunktion ist, größer als Null ist.

8. Computerlesbares Medium, das ein Programm enthält, das, wenn es geladen wird, ein Verfahren zum Bewerten eines Sterblichkeitsrisikos eines Herzpatienten auf der Grundlage von wenigstens einem Lebenszeichen oder Biosignal des Patienten durchführt, das die folgenden Schritte umfasst:
a. Auswählen, gemäß einer vorab festgelegten Routine, von wenigstens einer Datensequenz aus wenigstens einer Aufzeichnung eines Elektrokardiogramms (EKG), das als wenigstens eine Datenfunktion des wenigstens einen Lebenszeichens oder Biosignals des Patienten bereitgestellt wird, wobei die Datensequenz wenigstens einen Datenpunkt enthält, der mit einer ventrikulären Extrasystole korreliert, wobei das Programm, das in dem computerlesbaren Medium enthalten
ist, **gekennzeichnet ist durch:**
b. Berechnen einer spezifischen T-Wellen-Antwort auf eine ventrikuläre Extrasystole auf der Grundlage der wenigstens einen ausgewählten Datensequenz **durch** Berechnen des Quotienten der T-Wellen-Amplituden zwischen der ersten post-extrasystolischen T-Wellen-Amplitude und dem Mittel von T-Wellen-Amplituden, die der ersten post-extrasystolischen T-Wellen-Amplitude vorausgehen und/oder folgen.
c. Bewerten eines Sterblichkeitsrisikos des Patienten auf der Grundlage der berechneten T-Wellen-Antwort **durch** Zuweisen des Patienten zu einer Gruppe mit hohem Risiko, wenn die Anzahl von Quotienten aus T-Wellen-Amplituden, die laut Berechnung kleiner als 1 pro 30 Minuten Aufzeichnung der Datenfunktion ist, größer als Null ist.

## Revendications

1. Dispositif d'évaluation d'un risque de mortalité d'un patient cardiaque sur base d'au moins un signe vital ou biosignal dudit patient, ledit dispositif comprenant :
a. une unité de procuration de données configurée pour procurer au moins un enregistrement d'électrocardiogramme (ECG) comme au moins une fonction de données dudit au moins un signe vital ou biosignal dudit patient ;
b. une unité de traitement de données configurée pour traiter des données de ladite au moins une fonction de données pour évaluer un risque de mortalité dudit patient en mettant en oeuvre les actions suivantes :
i. sélection d'au moins une séquence de données à partir de ladite au moins une fonction de données conformément à une routine prédéterminée, dans lequel la séquence de données contient au moins un point de donnée corrélé avec une extrasystole ventriculaire,
ledit dispositif étant **caractérisé par :**
ii. le calcul d'une réponse d'onde T spécifique à une extrasystole ventriculaire sur base de ladite au moins une séquence de données sélectionnée en calculant le quotient d'amplitude d'onde T entre la première amplitude d'onde T post-extrasystolique et la moyenne des amplitudes d'onde T précédant et/ou suivant ladite première amplitude d'onde T post-extrasystolique ;
iii. l'évaluation d'un risque de mortalité dudit patient sur base de ladite réponse d'onde T calculée à une extrasystole ventriculaire en attribuant ledit patient à un groupe à haut risque dans le cas où le nombre de quotients d'amplitude d'onde T calculés comme étant inférieurs à 1 dans un enregistrement de 30 minutes de ladite fonction de données est supérieur à zéro.

2. Dispositif selon la revendication 1, **caractérisé en ce que** ladite unité de procuration de données est configurée pour mettre en oeuvre au moins l'une des actions suivantes :
a. enregistrement dudit au moins un signe vital ou biosignal, de préférence sous forme numérique ;
b. enregistrement dudit au moins un signe vital ou biosignal de manière continue durant une période prédéterminée, de préférence durant au moins 30 minutes ;
c. enregistrement dudit au moins un signe vital ou biosignal avec un moyen d'enregistrement non invasif, de préférence en utilisant le dispositif suivant :
i. un enregistreur d'électrocardiogramme, de préférence un enregistreur d'électrocardiogramme à haute résolution avec au moins 1,6 kHz dans des dérivations XYZ orthogonales, de préférence pour l'enregistrement en continu d'un électrocardiogramme dudit patient ;
d. enregistrement dudit au moins un signe vital ou biosignal dans une position de repos dudit patient, de préférence dans une position de repos en supination dudit patient ;
e. enregistrement simultané d'au moins deux signes vitaux ou biosignaux différents, de préférence un électrocardiogramme et une pression sanguine, de préférence une tension artérielle continue ;
f. stockage des données enregistrées dans un moyen de stockage de données, de préférence sous forme numérique ;
g. fourniture d'au moins une fonction de données sous forme d'une fonction temporelle ;
h. habilitation d'une vérification et/ou d'une révision et/ou d'une correction manuelle de ladite au moins une fonction de données, en particulier en habilitant la révision et/ou la correction manuelle de classifications QRS de manière à différentier des complexes prématurés sinusaux et ventriculaires (VPC) ;
i. chargement de ladite au moins une fonction de données à partir du moyen de stockage de données, de préférence sous forme numérique.

3. Dispositif selon l'une des revendications précédentes 1 et 2, **caractérisé en ce que** ladite unité de traitement de données est configurée pour sélectionner ladite au moins une séquence de données à partir de ladite au moins une fonction de données en mettant en oeuvre au moins l'une des actions suivantes :
a. identification de motifs périodiques au sein de ladite au moins une fonction de données, de préférence des complexes QRS ;
b. identification de points de données de ladite au moins une fonction de données qui sont corrélés avec :
i. des pics R des complexes QRS d'un électrocardiogramme ;
c. mesure des intervalles entre les points de chaque paire de points de données subséquents, de préférence entre deux pics R successifs de complexes QRS.
d. calcul d'un quotient entre un intervalle d'intérêt et un intervalle moyen, dans lequel l'intervalle moyen peut être calculé à partir d'au moins l'un des éléments suivantes :
i. un nombre d'intervalles consécutifs précédant et/ou suivant l'intervalle d'intérêt, dans lequel le nombre d'intervalles consécutifs est de préférence deux, trois, quatre, cinq, six, sept, huit, neuf ou dix ;
ii. un nombre d'intervalles consécutifs précédant et/ou suivant une extrasystole ventriculaire, dans lequel le nombre d'intervalles consécutifs est de préférence deux, trois, quatre, cinq, six, sept, huit, neuf ou dix ;
e. sélection d'une séquence de données pour un traitement ultérieur dans au moins l'un des cas suivants :
i. la séquence de données contient au moins un nombre de points de données consécutifs corrélés avec des systoles ventriculaires, régulières précédant et/ou suivant une extrasystole ventriculaire ;
ii. le quotient calculé pour au moins un intervalle répond à au moins un critère mathématique ;
iii. les quotients calculés pour au moins deux intervalles subséquents répondent à différents critères mathématiques ;
iv. les quotients calculés pour au moins deux intervalles subséquents sortent d'une plage de valeurs prédéterminée ;
v. le quotient calculé pour un premier intervalle de ladite séquence de données est inférieur ou égal à une première valeur et/ou le quotient calculé pour un deuxième intervalle subséquent au premier intervalle est supérieur ou égal à une deuxième valeur ;
vi. le quotient calculé pour un intervalle de ladite séquence de données est inférieur ou égal à une première valeur et/ou le quotient calculé pour un nombre d'intervalles consécutifs subséquents est supérieur ou égal à ladite première valeur.

4. Dispositif selon l'une des revendications précédentes 1 à 3, **caractérisé en ce que** ladite unité de traitement de données est configurée pour calculer une réponse d'onde T à une extrasystole ventriculaire sur base de ladite séquence de données en mettant en oeuvre au moins l'une des actions suivantes :
a. stockage du quotient d'amplitude d'onde T calculé ou de la moyenne d'une pluralité de quotients d'amplitude d'onde T calculés sous la variable PEST ;
b. stockage du nombre d'amplitudes d'onde T calculées, de préférence en fonction du temps.

5. Dispositif selon l'une des revendications précédentes 1 à 4, **caractérisé en ce que** ladite unité de traitement de données est configurée pour évaluer un risque de mortalité pour ledit patient en procurant au moins l'une des fonctions suivantes :
a. attribution dudit patient à un groupe à faible risque sous les conditions suivantes :
i. le nombre de séquences de données sélectionnées par enregistrement de 30 minutes de ladite au moins une fonction de données est inférieur à cinq ; et
ii. le nombre de quotients d'amplitude d'onde T calculés comme étant inférieurs à 1 dans un enregistrement de 30 minutes de ladite au moins une fonction de données est égal à zéro ;
b. attribution dudit patient à un groupe à risque moyen sous les conditions suivantes :
i. le nombre de séquences de données sélectionnées par enregistrement de 30 minutes de ladite au moins une fonction de données est supérieur à cinq ; et
ii. le nombre de quotients d'amplitude d'onde T calculés comme étant inférieurs à 1 dans un enregistrement de 30 minutes de ladite au moins une fonction de données est égal à zéro ;
c. attribution dudit patient à un groupe à haut risque sous au moins l'une des conditions suivantes :
i. le nombre de séquences de données sélectionnées par enregistrement de 30 minutes de ladite au moins une fonction de données est supérieur à cinq ;
ii. le PEST est inférieur à un ;
d. évaluation du risque de mortalité dudit patient en calculant un score de pronostic sur base d'au moins l'un des paramètres suivants :
i. PEST ;
ii. VPC ;
iii. turbulence de rythme cardiaque HRT, soit Heart Rate Turbulence, en particulier HRT-TS ≤ 2, 5 ms/RRI et/ou HRT-TO ≥ 0 %.

6. Dispositif selon l'une des revendications précédentes 1 à 5, **caractérisé en ce que** ledit dispositif comprend un moyen d'affichage pour afficher le résultat d'une évaluation de risque de mortalité.

7. Procédé d'évaluation d'un risque de mortalité d'un patient cardiaque sur base d'au moins un signe vital ou biosignal dudit patient, ledit procédé comprenant les étapes suivantes :
a. fourniture par une unité de procuration de données d'au moins un enregistrement d'électrocardiogramme (ECG) comme au moins une fonction de données dudit au moins un signe vital ou biosignal dudit patient ;
b. traitement par une unité de traitement de données de données de ladite au moins une fonction de données pour évaluer un risque de mortalité dudit patient en mettant en oeuvre les actions suivantes :
i. sélection d'au moins une séquence de données à partir de ladite au moins une fonction de données conformément à une routine prédéterminée, dans lequel la séquence de données contient au moins un point de donnée corrélé avec une extrasystole ventriculaire,
ledit procédé étant **caractérisé par :**
ii. le calcul d'une réponse d'onde T spécifique à une extrasystole ventriculaire sur base de ladite au moins une séquence de données sélectionnée en calculant le quotient d'amplitude d'onde T entre la première amplitude d'onde T post-extrasystolique et la moyenne des amplitudes d'onde T précédant et/ou suivant ladite première amplitude d'onde T post-extrasystolique ;
iii. l'évaluation d'un risque de mortalité dudit patient sur base de ladite réponse d'onde T calculée en attribuant ledit patient à un groupe à haut risque dans le cas où le nombre de quotients d'amplitude d'onde T calculés comme étant inférieurs à 1 dans un enregistrement de 30 minutes de ladite fonction de données est supérieur à zéro.

8. Support lisible par un ordinateur contenant un programme qui, lorsqu'il est chargé, met en oeuvre un procédé d'évaluation d'un risque de mortalité d'un patient cardiaque sur base d'au moins un signe vital ou biosignal dudit patient, comprenant les étapes suivantes :
a. sélection, conformément à une routine prédéterminée, d'au moins une séquence de données parmi au moins un enregistrement d'électrocardiogramme (ECG) qui est fourni comme étant au moins une fonction de données dudit au moins un signe vital ou biosignal dudit patient, dans lequel la séquence de données contient au moins un point de donnée corrélé avec une extrasystole ventriculaire, ledit programme contenu dans ledit support lisible par un ordinateur étant **caractérisé par :**
b. le calcul d'une réponse d'onde T spécifique à une extrasystole ventriculaire sur base de ladite au moins une séquence de données sélectionnée en calculant le quotient d'amplitude d'onde T entre la première amplitude d'onde T post-extrasystolique et la moyenne des amplitudes d'onde T précédant et/ou suivant ladite première amplitude d'onde T post-extrasystolique ;
c. l'évaluation d'un risque de mortalité dudit patient sur base de ladite réponse d'onde T calculée en attribuant ledit patient à un groupe à haut risque dans le cas où le nombre de quotients d'amplitude d'onde T calculés comme étant inférieurs à 1 dans un enregistrement de 30 minutes de ladite fonction de données est supérieur à zéro.
